# EUROPEAN PATENT APPLICATION

(11) **EP 2 502 910 A1**
(43) Date of publication of application: **26.09.2012**
(21) Application number: 11158264.9
(22) Date of filing: 15.03.2011
(51) Int. Cl.: C07D 215/18, A61K 31/47, A61P 11/06

(54) **Camphorsulfonic salt of a key Montelukast intermediate**

(71) Applicant: Laboratorios Lesvi, S.L., 08970 Sant Joan Despí - Barcelona (ES)
(72) Inventor: Dalmases Barjoan, Pere, E-08970, Sant Joan Despí - Barcelona (ES); Ozores Viturro, Lidia, E-08970, Sant Joan Despí - Barcelona (ES)
(74) Representative: ABG Patentes, S.L.

(57) **Abstract**

The present invention is directed to a novel salt of a montelukast intermediate, the process of preparation thereof, and use of such salt in the preparation of sodium montelukast via a new amine salt of montelukast.

## Description

### FIELD OF THE INVENTION

The present invention is directed to a novel salt of a montelukast intermediate, the process of preparation thereof, and use of such salt in the preparation of sodium montelukast via a new amine salt of montelukast.

### BACKGROUND

Montelukast or (1-{1-(*R*)-(*E*)-{3-[2-(7-chloro-quinolin-2-yl)-vinyl]-phenyl}-3-[2-(1-hydroxy-1-methyl-ethyl)-phenyl]-propylsulfanylmethyl}-cyclopropyl)-acetic acid has the following formula (5).

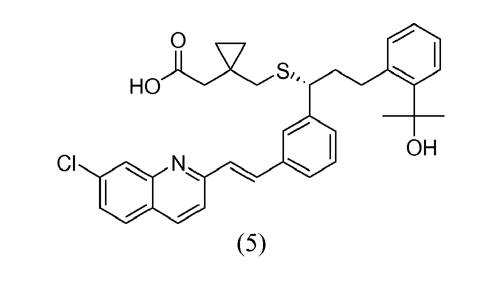

The sodium salt of montelukast is marketed under the name of SINGULAIR® (Merck) administered in an oral form for the treatment of asthma.

EP 0 480 717 B1 described for the first time a family of compounds comprising sodium montelukast. The preparation process to obtain said compounds involved the use of methyl esters, such as methyl 2-[(3S)-[3-[(2E)-(7-chloroquinolyn-2-yl)ethenylphenyl]-3-hydroxipropyl]benzoate (6), comprising the coupling between methyl 1-(mercaptometyl)-cyclopropaneacetate (9) and an appropriate mesylate (8), produced *in situ.* The methyl ester of montelukast (10) was hydrolyzed into its acid and directly transformed into its corresponding sodium salt (11), as shown in Scheme 1. Tedious chromatographic purifications of the methyl esters and the final products made the aforementioned process unsuitable for large scale production. In addition, the yields obtained were poor and sodium montelukast was obtained as an oily substance. Sodium montelukast can also be obtained in an amorphous form by lyophilisation; however this production method is significantly uneconomical at industrial scale.

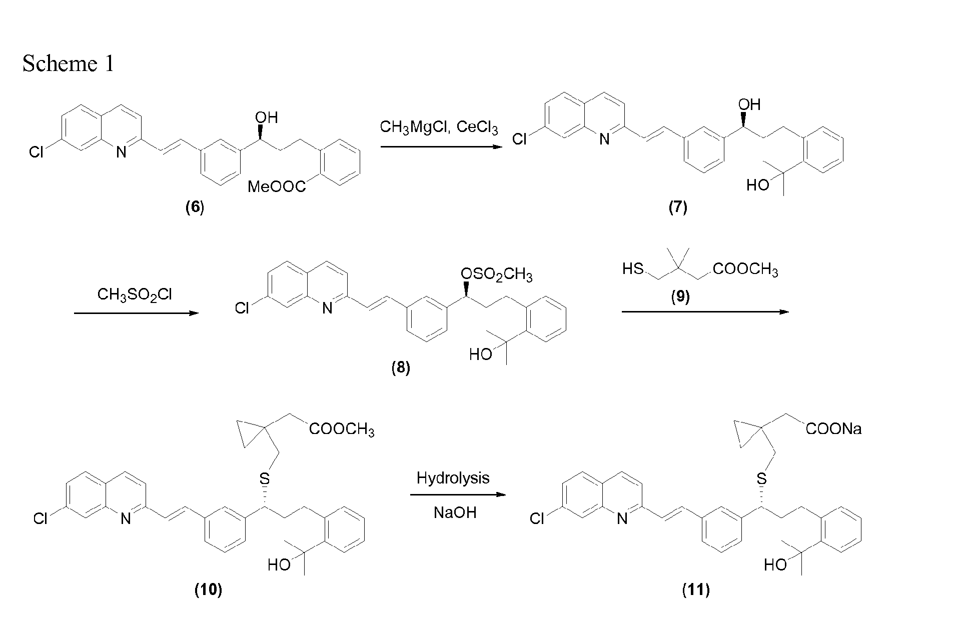

EP 0 737 186 B1 described an improved process for the synthesis of sodium montelukast (Scheme 2), which differed from the process described in EP 0 480 717 B1 in the use of the dilithium salt of 1-(mercaptomethyl)cyclopropaneacetic acid, instead of the methyl ester, for the coupling reaction with the mesylate. Said mesylate (8) had the same formula as in EP 0 480 717 B1 but was added in its crystalline form. The process directly yielded montelukast in its acid form, which was further transformed into a crystalline dicyclohexylamine salt (13). The purified and crystalline dicyclohexylamine salt (13) was treated with an acid in order to obtain montelukast in its acid form, which was further treated with a source of sodium ions to yield the sodium salt (11).

The procedure disclosed in EP 0 737 186 B1 showed some disadvantages such as the requirement of some steps to be performed at temperatures of about -25°C and below. Furthermore, some of the intermediates used, such as the mesylate, are unstable. Moreover, highly flammable and hazardous chemicals, such as butyl lithium, were used. The above disadvantages make the process not convenient for industrial scale applications.

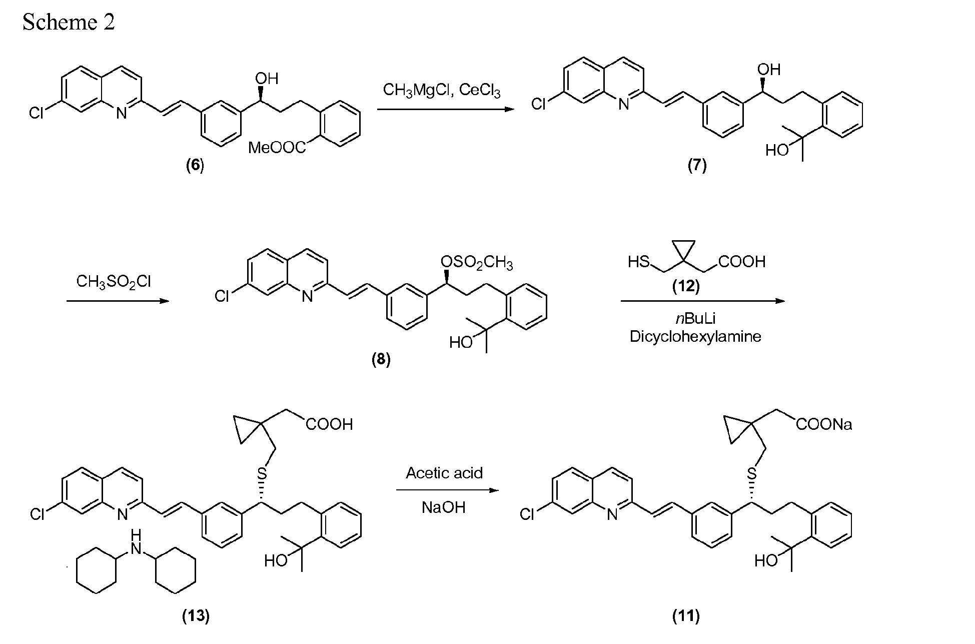

CN 1420113 A describes an alternative process for the preparation of montelukast (Scheme 3), wherein treatment of methyl 2-((3R)-acetylsulfanyl)-3-[3-[(*E*)-2-(7-chloro-2-quinolinyl)-ethenyl]-phenyl]-propyl)-benzoate (14) with an excess of methylmagnesium halide affords 2-[2-(3-{3-[2-(7-chloro-quinolin-2-yl)-vinyl]-phenyl}-3-mercapto-propyl)-phenyl]-propan-2-ol (3). The main drawback of this process is the formation of a ketone impurity (15) in considerably large amounts.

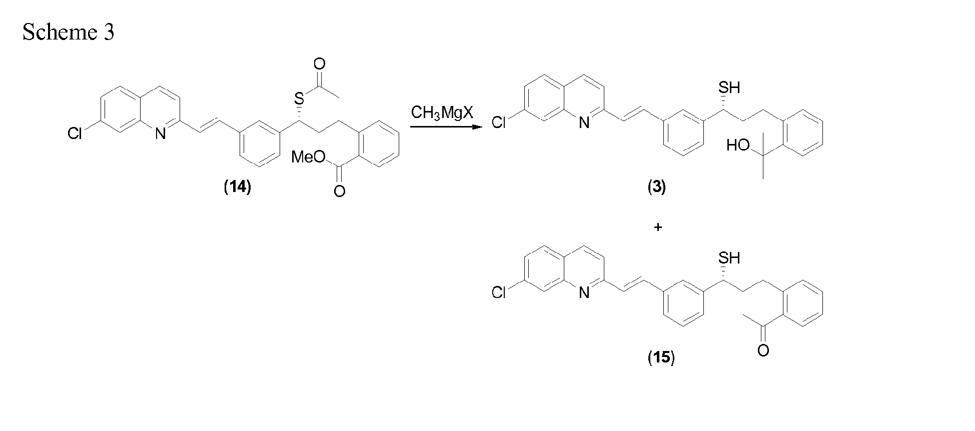

Patent applications US 2009/0093636 A1, WO 2007/057228 A1, WO 2007/057227 Al and WO 2007/057225 Al describe a process (Scheme 4), which involves the reaction between methyl 2-((3R)-acetylsulfanyl)-3-[3-[(*E*)-2-(7-chloro-2-quinolinyl)-ethenyl]-phenyl]-propyl)-benzoate (14) and a methylmagnesium halide to afford a cyclic intermediate (16), (3R)-{3-[(E)-2-(7-chloro-2quinolinyl)-vinyl]-phenyl}-4,5-dihydro-3H-benzo[c]thiepin-1-one, which can be isolated and purified. Afterwards, intermediate (16) is further treated with an excess of a methyl magnesium halide and an activated cerium (III) salt to afford 2-[2-(3- {3-[2-(7-chloro-quinolin-2-yl)-vinyl]-phenyl}-3-mercapto-propyl)-phenyl]-propan-2-ol (3). As a result of it, the amount of ketone impurity (15) is reduced. However compound (3) is unstable. To overcome this problem, patent application US 2009/0093636 Al describes the preparation of hydrochloride, tosylate and besylate salts, although only the tosylate salt is described in detail and the yield obtained (of about 50%) was very low.

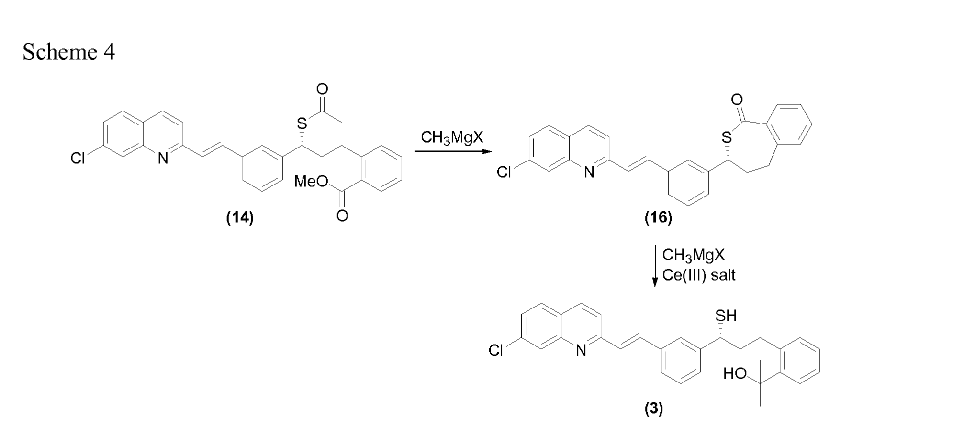

The inventors have surprisingly found a process whereby a new salt of compound (3) can be prepared in higher yields and in higher purity. The process allows the preparation of the camphorsulfonic salt of compound (3) having formula (1) depicted below.

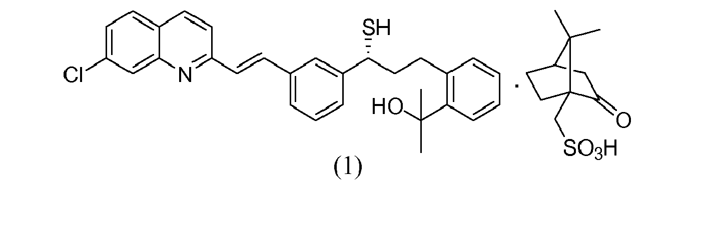

The salt of formula (1) is used in the synthesis of amorphous sodium montelukast via formation of a new diisobutylamine salt of montelukast, as shown in the examples of the present patent application. This process avoids tedious chromatographic purification and non-economical lyophilisation providing an alternate way to make sodium montelukast, which would be suitable for large scale production.

Elimination or reduction of certain impurities is achieved when sodium montelukast is prepared using the canforsulfonate salt and the diisobutyl amine salt, which will be difficult to remove in the final step, of formation of sodium montelukast, due to the similarities in the structure of the impurities and the final product.

Moreover, this preparation process is also much faster than the one described in patent applications US 2009/0093636 A1, WO 2007/057228 A1, WO 2007/057227 Al and WO 2007/057225 A1.

### SUMMARY OF THE INVENTION

The first aspect of the present invention relates to 2-[2-(3-{3-[2-(7-chloroquinolin-2-yl)-vinyl]-phenyl}-3-mercapto-propyl)-phenyl]-propan-2-ol camphorsulfonate salt of formula (1).

In the second aspect, the invention provides a process for preparing said camphorsulfonate salt of formula (1) comprising: mixing a cerium (III) salt and tetrahydrofuran; adding a methylmagnesium halide to the previously obtained mixture; causing the mixture thus obtained to react with a 6-acetylsulfanyl-11-(7-chloroquinolin-2-yl)-2-ethylidene-7-methyl-3,9-dimethylene-undeca-7,10-dienoic acid C₁-C₃ alkyl ester of formula (2); treating the mixture thus obtained with camphorsulfonic acid of formula (4); and isolating the salt of formula (1).

The third aspect of the invention relates to the use of said camphorsulfonate salt of formula (1) for the preparation of sodium montelukast

A fourth aspect of the invention relates to use of camphorsulfonate salt of formula (1) for the preparation of new diisobutyl salt of montelukast.

A fifth aspect of the invention relates a new diisobutyl amine salt of montelukast.

A sixth aspect of the invention relates to the use of the diisobutylamine salt of montelukast in the preparation of sodium montelukast.

### DESCRIPTION OF THE FIGURES

Figure 1 shows the infrared (IR) spectrum of the salt of formula (1).
Figure 2 shows the X-ray diffraction pattern (XRD) of the salt of formula (1).
Figure 3 shows the infrared (IR) spectrum of montelukast diisobutylamine salt (19).
Figure 4 shows the X-ray diffraction pattern (XRD) of montelukast diisobutylamine salt (19).
Figure 5 shows the infrared (IR) spectrum of sodium montelukast (11).

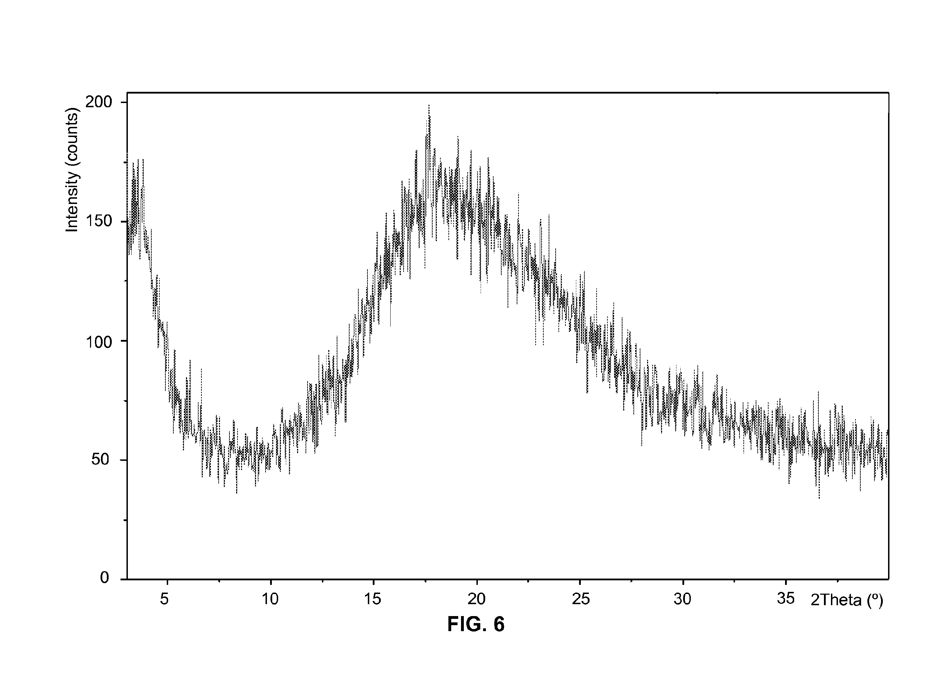

Figure 6 shows the X-ray diffraction pattern (XRD) of sodium montelukast (11).

### DESCRIPTION OF THE INVENTION

### Salt of formula (1)

According to the first aspect, the present invention is directed to a novel salt of formula (1), which is the camphorsulfonate salt of the compound of formula (3). The compound of formula (3) is a key intermediate in the synthesis of montelukast, however compound of formula (3) is unstable. The inventors have discovered the new stable camphorsulfonic salt of formula (1), which is obtained in high yield and purity.

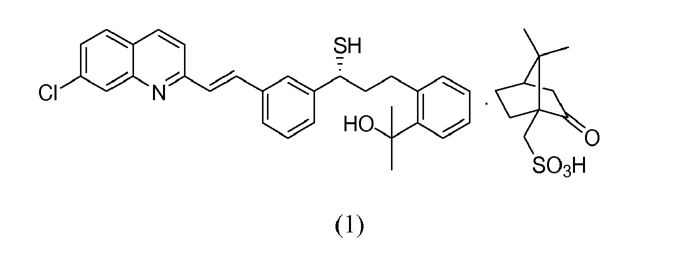

Camphorsulfonic acid can be used in any of its enantiomeric forms or mixtures thereof to give the corresponding diastereomeric salt, i.e. (R)-camphorsulfonate salt, (S)-camphorsulfonate salt, or mixtures thereof. In a particular embodiment, the camphorsulfonic acid is used in the form of the (R)-enantiomer yielding the (R)-camphorsulfonate salt.

### Process for the preparation of the salt of formula (1)

According to the second aspect, the invention provides a method for the preparation of camphorsulfonic salt of formula (1), which comprises:
a) mixing a cerium (III) salt and tetrahydrofuran (THF);
b) adding a methylmagnesium halide to the mixture of step a);
c) causing the mixture of step b) to react with a compound of formula (2), 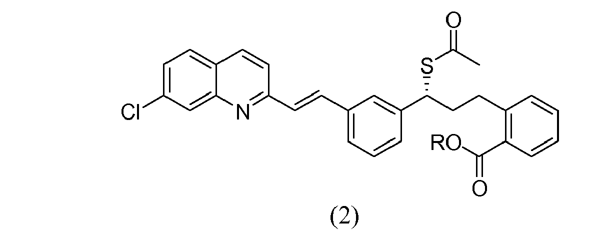 wherein R is C₁-C₃ alkyl group;
d) treating the reaction product of step c) with an aqueous acetic acid
   solution to yield a compound of formula (3) 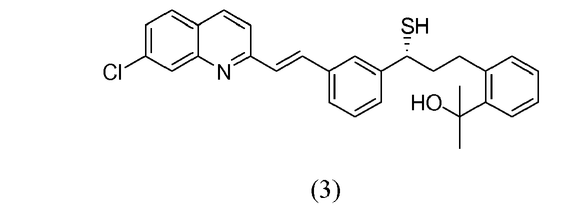
e) treating a mixture of the compound of formula (3) in an organic solvent
   with camphorsulfonic acid of formula (4); and 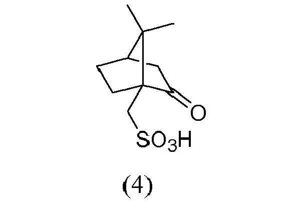
f) isolating the salt of formula (1).

The following Scheme 5 depicts the process described above.

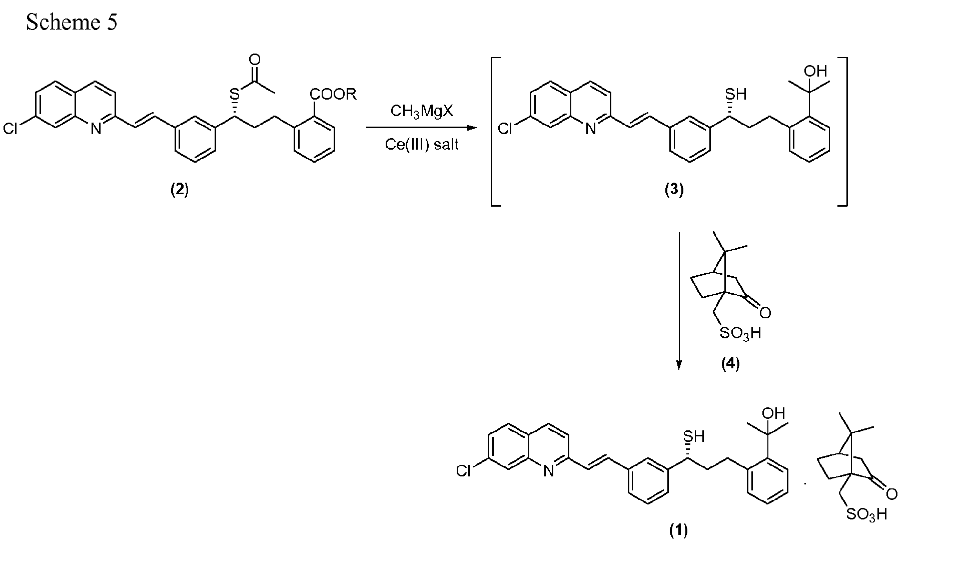

The compound of formula (2), wherein R is a C₁-C₃ alkyl group, is used as starting compound for the preparation of the salt of formula (1) in the present invention. According to a particular embodiment the compound of formula (2) is the methyl ester, wherein R is a methyl group. This compound can be prepared according to the method disclosed in US patent application US 2005/0245568 A1.

According to the process described above, the first step in the preparation of the salt of formula (1) is the activation of the cerium (III) salt, which is achieved by mixing the cerium (III) salt and tetrahydrofuran. A process for the activation of cerium chloride has been described by Conlon et al [Conlon, D., Kumke, D., Moeder, C., Hardiman, M., Hutson, G. and Sailer, L., Insights into the Cerium Chloride-Catalyzed Grignard Addition to Esters. Advanced Synthesis & Catalysis, 2004, 346,1307―1315].

According to an embodiment of the invention, such activation carried out by maintaining the mixture of the cerium (III) salt and tetrahydrofuran at a temperature comprised between 20 and 70 °C for a time period of 30 to 240 minutes and then cooling to a temperature comprised between -20 °C and 0 °C prior to the addition of a methylmagnesium halide.

The term methylmagnesium halide refers to methylmagnesium chloride, bromide or iodide. According to a particular embodiment of the invention, the methylmagnesium halide is methylmagnesium chloride.

In another embodiment of the invention, the cerium (III) salt is CeCl₃, preferably CeCl₃ containing less than 2% water by weight, even more preferably CeCl₃ containing less than 1% water by weight.

Preferably, the methylmagnesium chloride with the mixture of CeCl₃ and tetrahydrofuran is stirred for a period of time comprised between 1 and 3 hours, prior to addition of the compound of formula (2) wherein R is a methyl group.

Preferably, the methylmagnesium chloride is added to the mixture of cerium (III) salt and tetrahydrofuran as a tetrahydrofuran solution.

The next step in the process is the reaction of compound of formula (2) with the mixture resulting from the activation of the cerium (III) salt and addition of methylmagnesium halide. Preferably, the compound of formula (2) is dissolved in a suitable organic solvent. Accordingly the organic solvent may be for example, an aromatic hydrocarbon, preferably toluene; an ester, such as ethyl acetate and isopropyl acetate; and ether, such as methyl tert-butyl ether; dichloromethane; or mixtures thereof.

The following step in the process is treatment of the mixture obtained in the previous step (resulting from reaction of the compound of formula (2) with the activated cerium (III) salt and the methylmagnesium halide) with an aqueous acetic acid solution to afford the compound of formula (3).

Finally, the reaction mixture resulting from the previous step is then treated with camphorsulfonic acid to provide the salt of formula (1). According to an embodiment of the invention, the camphorsulfonic acid used for obtaining the salt of formula (1) is (R)-camphorsulfonic acid. Preferably, this acid is dissolved in an organic solvent. Accordingly the organic solvent may be for example, an aromatic hydrocarbon, preferably toluene; an ester, such as ethyl acetate and isopropyl acetate; and ether, such as tetrahydrofuran, 2-methyltetrahydrofuran and methyl tert-butyl ether; dichloromethane; or mixtures thereof.

In an embodiment of the invention the ratio of camphorsulfonic acid to the compound of formula (2) is comprised between 0.9:1 and 1.2:1.

Camphorsulfonic acid can be used in any of its enantiomeric forms to give the corresponding salt, i.e. (R)-camphorsulfonate salt of formula (1), (S)-camphorsulfonate salt, or racemic camphorsulfonate salt.

The salt of formula (1) obtained according to the process described above can be isolated by conventional separating methods, such as filtration, decantation, centrifugation, precipitation, crystallisation. Preferably, the salt of formula (1) is isolated by filtration.

Said salt (1) is obtained in high purity and thus there is no need for further purification steps.

Surprisingly, the process described above for the synthesis of the salt of formula (1) offers a simple and efficient method for the preparation of a stable salt of the compound of formula (3), resulting in higher yields and fewer impurities.

### Use of the salt of formula (1) in a process for the preparation of sodium montelukast

The third aspect of the present invention relates to the use of the salt of formula (1) in a process for the preparation of sodium montelukast, as shown in Scheme 5. Preferably, sodium montelukast is prepared in amorphous form.

According to an embodiment of the invention, the process for the preparation of sodium montelukast using the salt of formula (1) comprises the following steps:
a) mixing the salt of formula (1) with an organic solvent;
b) optionally treating the mixture of step a) with a weak base;
c) adding (1-halomethyl-cyclopropyl)-acetic acid C₁-C₃ alkyl ester and a strong base to the product obtained in step a) or b);
d) hydrolyzing the ester obtained in step c) with a strong base;
e) acidifying the reaction mixture of step d)
f) treating the product obtained in step e) with a primary or secondary organic amine;
g) isolating montelukast amine salt;
h) mixing the montelukast amine salt with an organic solvent;
i) treating the product thus obtained with a source of sodium ion; and
j) isolating sodium montelukast.

The following Scheme 6 depicts the process described above.

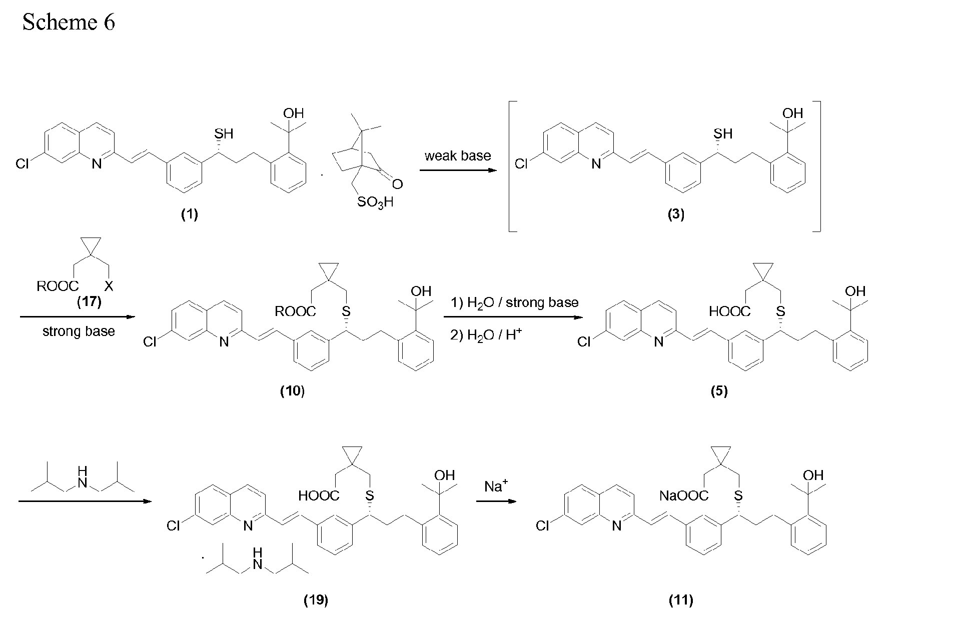

The first step (step a) of this process (Scheme 6) is mixing the salt of formula (1) with an organic solvent. Accordingly the organic solvent may be for example, an aromatic hydrocarbon, preferably toluene; an ester, such as ethyl acetate and isopropyl acetate; an ether, such as tetrahydrofuran, 2-methyltetrahydrofuran and methyl *tert-*butyl ether; dichloromethane; an alcohol, such as methanol; dimethylformamide (DMF); or mixtures thereof.

The next step (step b) of this process (Scheme 6) is optional, and is the recovery of the free base of the salt of formula (1). Said step is carried out by treating the salt of formula (1) with a weak base.

The term weak base refers to base wherein the pKₐ (relative to water) of the corresponding conjugate acid is comprised between 5 and 11. Preferably the base is inorganic. Examples of such bases are alkali metal or alkaline earth metal hydrogen carbonates, such as NaHCO₃; alkali metal or alkaline earth metal carbonates, such as Na₂CO₃, K₂CO₃; alkali metal or alkaline earth metal sulfites, such as Na₂SO₃. The weak base is in the form of an aqueous solution. Preferably the weak base is NaHCO₃.

When the process includes optional step b) consisting of the treatment with a base, the resulting mixture is extracted with an organic solvent such as aromatic hydrocarbon, preferably toluene; an ester, such as ethyl acetate and isopropyl acetate; a water insoluble ether, such as methyl *tert*-butyl ether; dichloromethane; or mixtures thereof to recover thiol (3).

The next step (step c) in the process (Scheme 6) is the addition of (1-halomethyl-cyclopropyl)-acetic acid C₁-C₃ alkyl ester (17) and a strong base to the solution of thiol (3) in an organic solvent of step b) or to the mixture of step a) containing product (1).

The addition of (1-halomethyl-cyclopropyl)-acetic acid C₁-C₃ alkyl ester (17) and the strong base may be performed in any order, for instance first (1-halomethyl-cyclopropyl)-acetic acid C₁-C₃ alkyl ester is added and then the strong base, or first the strong base is added and then (1-halomethyl-cyclopropyl)-acetic acid C₁-C₃ alkyl ester, or (1-halomethyl-cyclopropyl)-acetic acid C₁-C₃ alkyl ester and the strong base are added simultaneously.

The halo refers to any of F, Cl, Br or I atoms, preferably a Br atom. The C₁-C₃ alkyl ester is preferably methyl, ethyl or propyl ester, even more preferably methyl. According to a particular embodiment of the invention, the (1-halomethyl-cyclopropyl)-acetic acid C₁-C₃ alkyl ester is (1-bromomethyl-cyclopropyl)-acetic acid methyl ester.

The term strong base refers to a base wherein the pKₐ (relative to water) of the corresponding conjugate acid is comprised between 11 and 16. Examples of such bases are alkali metal alkoxides, such as sodium methoxide, potassium methoxide and potassium propoxide; or alkali metal hydroxides, such as sodium hydroxide and lithium hydroxide. According to a particular embodiment of the invention, the alkali metal alkoxide is sodium methoxide.

Then (step d), montelukast free acid (5) is obtained by treatment of the ester thioether compound (10), obtained in the previous step, with a mixture of an organic solvent and water catalyzed by a strong base (Scheme 6), wherein the pKₐ (relative to water) of the corresponding conjugate acid is comprised between 11 and 16, followed by conventional acid work-up (step e), wherein the pKₐ (relative to water) of the acid is lower than 10, such as aqueous acid treatment, and isolation of the organic phase. Suitable bases include but are not limited to hydroxides of alkali metals or alkaline earth metals. In a preferred embodiment of the invention sodium hydroxide is used. Preferably, these bases are in aqueous solution. Subsequent acid work-up (step e) affords montelukast free acid. Suitable acids include but are not limited to weak acids, such as citric acid, acetic acid, boric acid, carbonic acid, phosphoric acid and hydrogen sulphide; preferably citric acid. In a particular embodiment of the invention, the ester thioether compound is methyl ester (10).

The next step (step f) in the process (Scheme 6) comprises treating montelukast free acid (5) with a primary or secondary organic amine, preferably diisobutylamine, in an organic solvent and isolating montelukast amine salt, preferably montelukast diisobutylamine salt (19). According to a preferred embodiment, the organic solvent is selected from an aromatic hydrocarbon, such as toluene; an ester such as ethyl acetate or isopropyl acetate; an ether such as tetrahydrofuran, 2-methyltetrahydrofuran or methyl *tert*-butyl ether; dichloromethane; dimethylformamide (DMF); or a mixture thereof. Preferably, said organic solvent is ethyl acetate.

In an embodiment, once montelukast amine salt is obtained, preferably montelukast diisobutylamine salt (19), it is further purified, for example by conventional methods, such as crystallization, or digestion. Election of the most suitable solvent requires only routine experimentation for the skilled person.

Finally, treatment of montelukast amine salt (Scheme 6), preferably montelukast diisobutylamine salt (19), with a source of sodium ion in an organic solvent affords sodium montelukast (11). Accordingly, the organic solvent may be for example, an aliphatic hydrocarbon, preferably methylcyclohexane; an aromatic hydrocarbon, preferably toluene; an ester such as ethyl acetate or isopropyl acetate; an ether such as tetrahydrofuran, 2-methyltetrahydrofuran or methyl *tert*-butyl ether; dichloromethane; or mixtures thereof. Accordingly the source of sodium ion is a salt, which can provide a sodium cation. Suitable sources of sodium ion may be, for example, sodium alkoxides, such as sodium methoxide, sodium ethoxide, sodium *tert*-butoxide, or sodium *tert-*pentoxide; sodium hydroxide; or mixtures thereof. Preferably, the source of sodium ion is selected from sodium *tert*-pentoxide. Sodium montelukast (11) can be isolated by filtration. Said compound is obtained in high purity and thus there is no need for further purification steps.

A further aspect of the invention relates to the use of the diisobutylamine salt of montelukast in the preparation of sodium montelukast (11). This preparation is achieved as explained above, i.e. by treatment of montelukast diisobutylamine salt (19) with a source of sodium ion in an organic solvent. Accordingly, the organic solvent may be for example, an aliphatic hydrocarbon, preferably methylcyclohexane; an aromatic hydrocarbon, preferably toluene; an ester such as ethyl acetate or isopropyl acetate; an ether such as tetrahydrofuran, 2-methyltetrahydrofuran or methyl *tert*-butyl ether; dichloromethane; or mixtures thereof. Accordingly the source of sodium ion is a salt, which can provide a sodium cation. Suitable sources of sodium ion may be, for example, sodium alkoxides, such as sodium methoxide, sodium ethoxide, sodium *tert-*butoxide, or sodium *tert*-pentoxide; sodium hydroxide; or mixtures thereof. Preferably, the source of sodium ion is sodium *tert*-pentoxide. Sodium montelukast (11) can be isolated by filtration. Said compound is obtained in high purity and thus there is no need for further purification steps.

### Definitions

In order to facilitate the comprehension of the present invention, the meanings of some terms and expressions as used in the context of the invention are included herein.

"Alkyl" refers to a hydrocarbon chain radical consisting of carbon and hydrogen atoms, linear or branched, containing no unsaturation, which is attached to the rest of the molecule by a single bond.

"Halide" means fluoride, chloride, bromide or iodide.

"Alkoxide" refers to an anion of the formula C₁-C₆ alkyl―O⁻, wherein alkyl has been previously defined, e. g. methoxide, ethoxide, propoxide, etc.

The term "aliphatic hydrocarbon" is a broad term that is used herein in its ordinary sense, as understood by those skilled in the art, and thus include acyclic or cyclic, saturated or unsaturated, linear or branched organic liquid compounds, excluding aromatic compounds, consisting exclusively of the elements carbon and hydrogen.

The term "aromatic hydrocarbon" is a broad term that is used herein in its ordinary sense, as understood by those skilled in the art, and thus include organic liquid compounds, which have at least one ring having a conjugated pi electron system, consisting exclusively of the elements carbon and hydrogen.

The term "alkali metal" refers to the Group 1 metals of the Periodic Table of the Elements, and comprises elements such as sodium, potassium and cesium.

The term "alkaline earth metal" refers to the Group 2 metals of the Periodic

Table of the Elements, and comprises elements such as beryllium, magnesium, calcium, strontium, and barium.

The term "weak base" refers to base wherein the pKₐ of the corresponding conjugate acid is comprised between 6 and 9. Example of such bases is NaHCO₃

The term "strong base" refers to a base wherein the pKₐ of the corresponding conjugate acid is comprised between 10 and 16. Examples of such bases are hydroxides of alkali metals, alkaline earth metals and alkali metal alkoxides.

### EXAMPLES

The following examples are displayed to illustrate the process of the present invention. They do not intend to limit in any way the scope of the invention defined in the present description.

### Example 1

### Preparation of the compound 2-[2-(3-{3-[2-(7-chloro-quinolin-2-yl)-vinyl]-phenyl}-3-mercapto-propyl)-phenyl]-propan-2-ol camphorsulfonate salt (1)

55.17 g of CeCl₃ and 462 mL of THF were mixed in a reactor and stirred at reflux for 2 hours. Afterwards, the mixture was cooled down to -20°C and 250 mL of a 3M solution of MeMgCl in THF were added, dropwise, during 45min. The reaction was stirred for 1 hour at 20-25 °C and cooled down to -20 °C.

A solution of 77 g of 6-Acetylsulfanyl-11-(7-chloro-quinolin-2-yl)-2-ethylidene-7-methyl-3,9-dimethylene-undeca-7,10-dienoic acid methyl ester (R=Me in formula 2) in 154 mL of toluene was added dropwise to the reactor. When the addition was completed, the solution was further stirred for 30min at -20 °C. Afterwards, 500.5 mL of an aqueous solution of acetic acid 2M were added and the formed phases were separated. In addition, the organic phase was washed with 192 mL of an aqueous solution of sodium carbonate 10% to give 2-[2-(3-{3-[2-(7-chloro-quinolin-2-yl)-vinyl]-phenyl}-3-mercapto-propyl)-phenyl]-propan-2-ol (3) dissolved in toluene and THF.

Afterwards, 33.5 g of (*R*)-camphorsulfonic acid dissolved in 141.5 mL of THF were charged into the reactor containing compound (3), followed by a quick formation of a precipitate. The resultant dispersion was stirred at room temperature for 2 hours, cooled down to 0-5 °C and further stirred for 30 minutes. The obtained precipitate was filtered and dried at 40 °C to give 102.92 g of 2-[2-(3-{3-[2-(7-chloro-quinolin-2-yl)-vinyl]-phenyl}-3-mercapto-propyl)-phenyl]-propan-2-ol camphorsulfonate salt (1).

### Yield: 97.65%. Purity (HPLC):97.75%

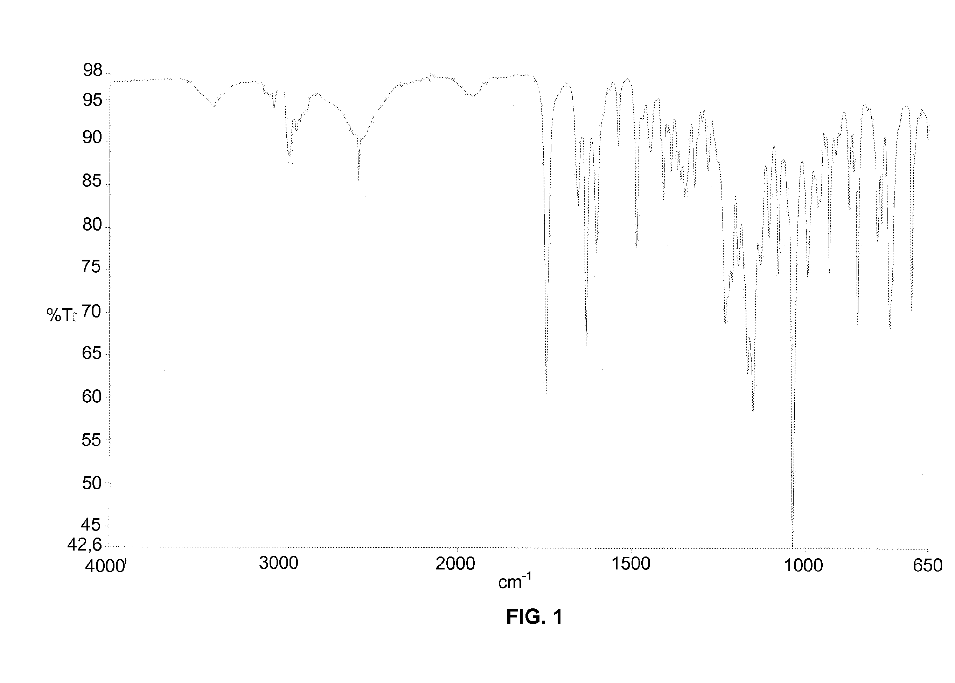

IR (cm⁻¹): 2960, 2566, 1744, 1654, 1630, 1601, 1539, 1487, 1448, 1409, 1386, 1348, 1320, 1281, 1230, 1193, 1167, 1151, 1106, 1079, 996, 966, 933, 876, 851, 793, 781, 757, 696, as shown in figure 1.

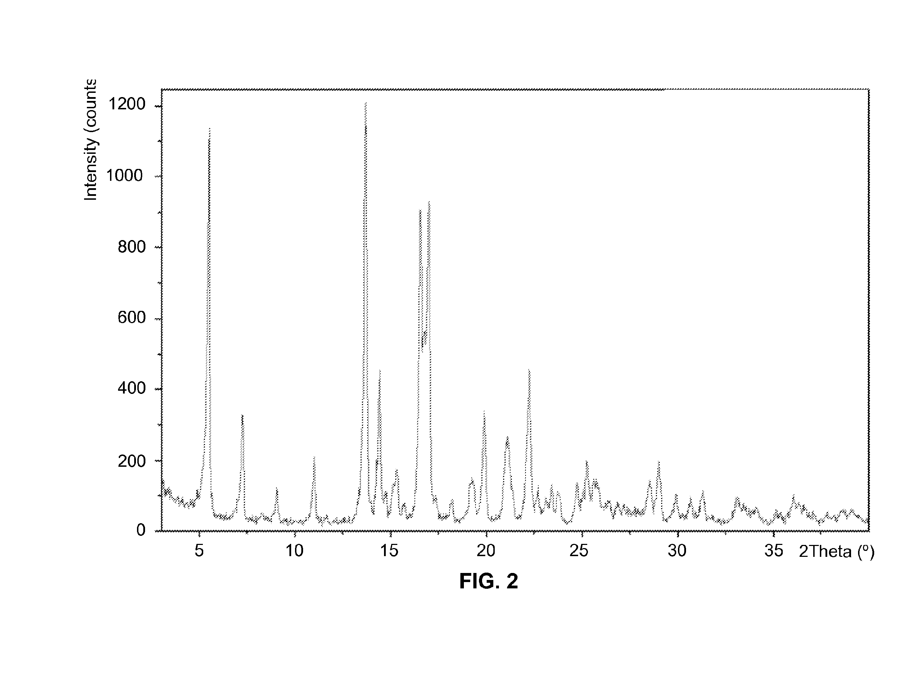

XRD (°2Th): 5.5, 7.3, 11.0, 13.7, 14.4, 15.3, 16.6, 17.0, 19.9, 21.1, 22.3, 25.3, 28.6 and 29.0, as shown in figure 2.

DSC: 195.36°C.

### Example 2

### Preparation of montelukast diisobutylamine salt (19)

228.2 g of 2-[2-(3-{3-[2-(7-chloro-quinolin-2-yl)-vinyl]-phenyl}-3-mercaptopropyl)-phenyl]-propan-2-ol camphorsulfonate (1), 559 mL of toluene, 186.8 mL of MeOH and 447 mL of a 5% aqueous solution of NaHCO₃ were charged in a reactor. The solution was stirred at 20-25 °C for 1 hour and once stirring was finalized, the different phases were separated.

The organic phase was set aside and cooled down to -15°C. Afterwards, 35.7 mL of NaOMe (30% in MeOH) and 35.8 g of (1-bromomethyl-cyclopropyl)-acetic acid methyl ester, previously dissolved in 22.3 mL of toluene, were added. The mixture obtained was further stirred for 30 minutes at -15 °C and for 14-18 h at 20-25 °C. Subsequently, the organic phase was extracted with 373 mL of citric acid 0.5 M and 75 mL of toluene. The extraction was carried out at 20-25 °C.

In addition, 112 mL of MeOH, 223 mL of THF and 135.3 mL of a 10% aqueous solution of NaOH were added into the reactor and stirred at 38-40°C for 1.5 hours. Subsequently, 75 mL of toluene and 372 mL of citric acid 0.5 M were added at a temperature of 0-5 °C. The mixture was stirred for 30 min at 20-25 °C and the phases were separated. Afterwards, 298 mL of AcOEt were added to the organic phase at 38-40 °C. The obtained suspension was protected from the light and maintained in an inert atmosphere.

Furthermore, 372 mL of AcOEt and 25.08 mL of diisobutylamine dissolved in 149 mL of AcOEt were added dropwise to the reactor at room temperature and stirred during 30 minutes. The solution was seeded and stirring was maintained for 16 hours and at 0-3 °C for 1.5 hours. The formed precipitate was filtered and washed with 150 mL of AcOEt to give 72.5g of montelukast diisobutylamine salt (19). The obtained solid was purified in 125 mL of AcOEt and 125 mL of toluene to yield crystalline montelukast diisobutylamine salt (19). Yield: 40%. Purity (HPLC): 99.34%.

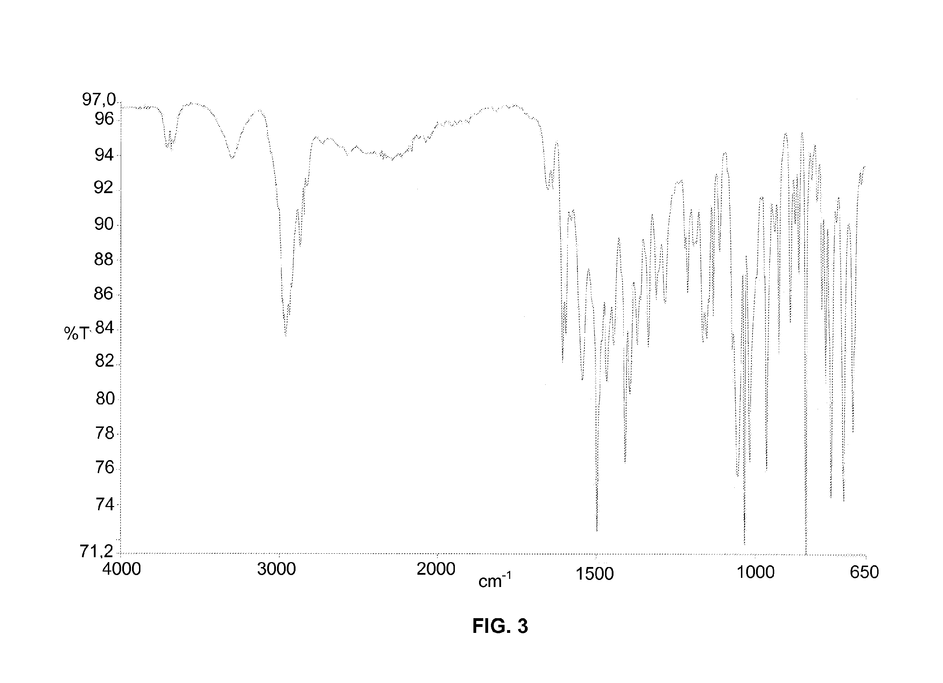

IR (cm⁻¹): 2959, 1606, 1544, 1497, 1467, 1409, 1394, 1371, 1336, 1284, 1212, 1164, 1131, 1111, 1054, 1033, 1017, 964, 925, 889, 862, 788, 777, 760, 720, 691, as shown in figure 3.

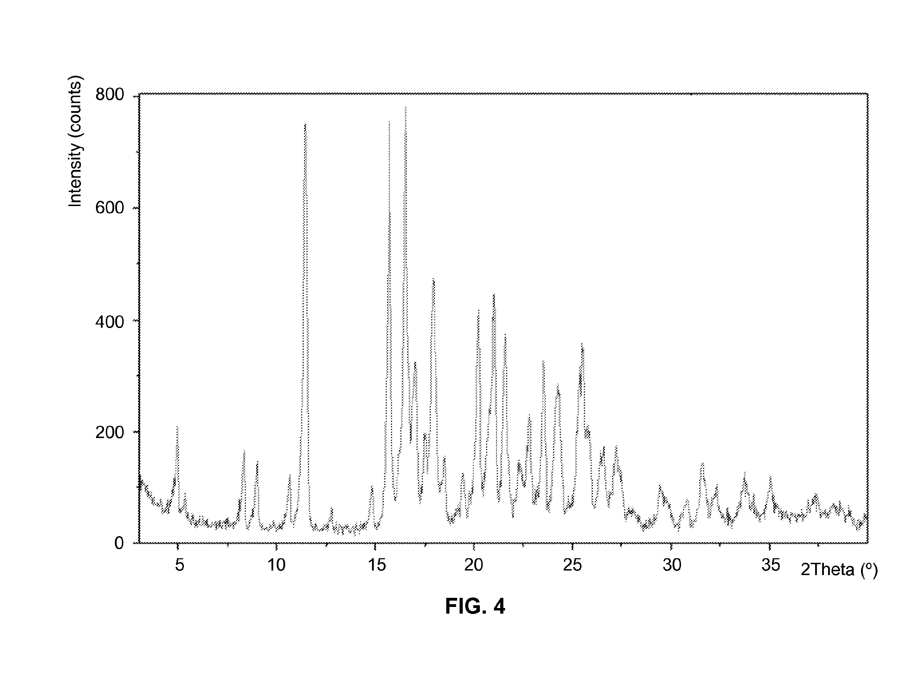

XRD (°2Th): 4.9, 11.4, 15.7, 16.5, 17.0, 17.5, 18.0, 18.1, 20.2, 21.0, 21.6, 22.8, 23.5, 24.3, 25.3, 25.5, 25.9, 26.6 and 27.2 as shown in figure 4.

DSC: 110.97 °C.

### Example 3

### Preparation of montelukast diisobutylamine salt (19)

44.9 g of 2-[2-(3-{3-[2-(7-chloro-quinolin-2-yl)-vinyl]-phenyl}-3-mercaptopropyl)-phenyl]-propan-2-ol camphorsulfonate (1) moist of toluene and THF, corresponding to 20.2 g of 2-[2-(3-{3-[2-(7-chloro-quinolin-2-yl)-vinyl]-phenyl}-3-mercapto-propyl)-phenyl]-propan-2-ol camphorsulfonate (1) dry, were mixed in a flask with 40 mL of DMF. In addition, 7.1 g of (1-bromomethyl-cyclopropyl)-acetic acid methyl ester were added and the mixture was cooled down to 0-5 °C. The reaction was carried out under an inert atmosphere using nitrogen as the inert agent. Afterwards, 14.2 mL of NaOMe (30% MeOH) were added and the reaction was mixed at 0-5 °C for 1 hour and a half. Subsequently, the organic phase was extracted with 100 mL of citric acid 0.5 M and 160 mL of toluene. The extraction was carried out at 20-25 °C.

In addition, 30 mL of MeOH, 60 mL of THF and 26.8 mL of a 10% aqueous solution of NaOH were added into the reactor and stirred at 38-40°C for 2.5 hours. A further extraction with 100 mL of citric acid 0.5 M and 40 mL of toluene was carried out at 20-25°C.

Furthermore, 80.8 mL of AcOEt and 5.03 mL of diisobutylamine dissolved in 40.4 mL of AcOEt were added dropwise to the reactor at room temperature and stirred during 16 hours at 20-25°C. The mixture was cooled down to 0-3 °C and further stirred for 2.5 hours. The formed precipitate was filtered and washed with 40 mL of AcOEt to yield 14.6 g of montelukast diisobutylamine salt (19). The obtained solid was crystallized to afford 10.2 g of montelukast diisobutylamine salt (19). Yield: 49.9%. Purity (HPLC): 99.54%.

### Example 4

### Preparation of montelukast sodium (11)

3 g of montelukast diisobutylamine (19), 15 mL of toluene and 486 mg of sodium tert-pentoxide were mixed and stirred at 40-45 °C for 30 minutes. 40.5 mL of methylcyclohexane were added dropwise and further stirred at room temperature for 1 hour. The obtained solid was filtered, washed with methycyclohexane and dried at 45 °C to give 1.43 g of montelukast sodium (11) in amorphous form. Yield: 56%. Purity (HPLC): 99.03%.

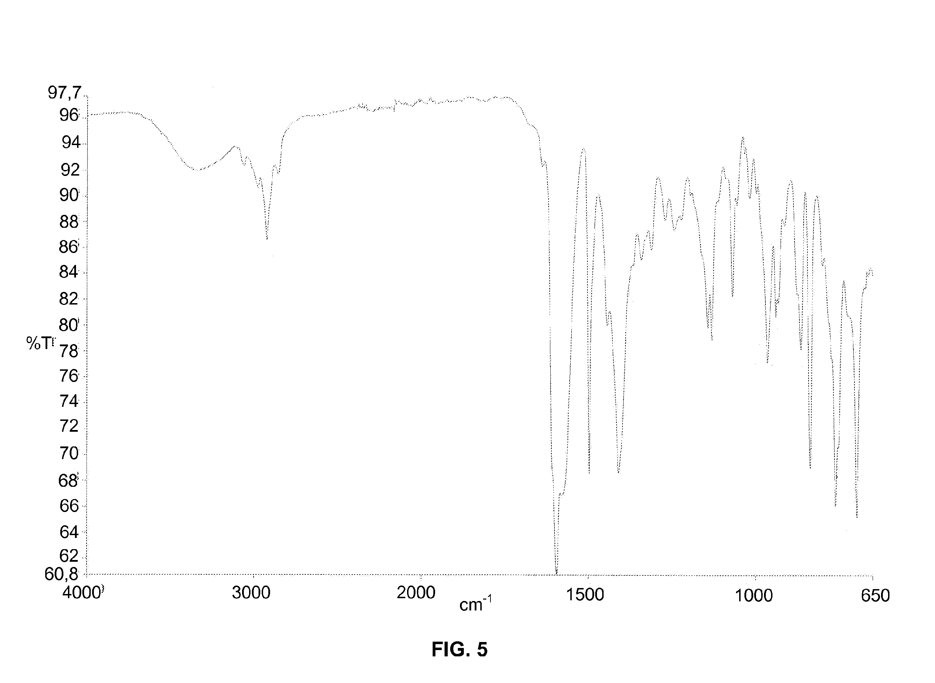

IR (cm⁻¹): 3351, 2921, 1497, 1442, 1408, 1340, 1312, 1270, 1242, 1143, 1131, 1068, 1017, 964, 958, 864, 836, 761, 697, as shown in figure 5.

## Claims

1. A salt of formula (1):

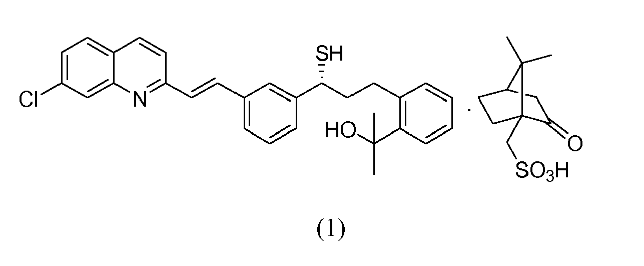

2. The salt according to claim 1, wherein the camphorsulfonic acid is (R)-camphorsulfonic acid.

3. A process for preparing the salt defined in anyone of claims 1 or 2 which comprises:
a) mixing a cerium (III) salt and tetrahydrofuran;
b) adding a methylmagnesium halide to the mixture of step a);
c) causing the mixture of step b) to react with a compound of formula (2), 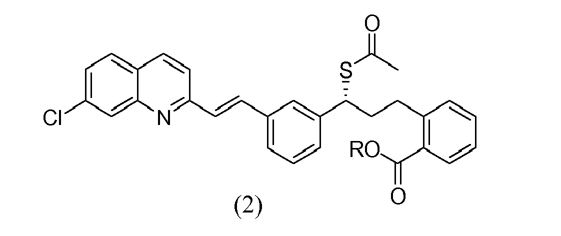 wherein R is C₁-C₃ alkyl group;
d) treating the reaction product of step c) with an aqueous acetic acid solution to yield a compound of formula (3) 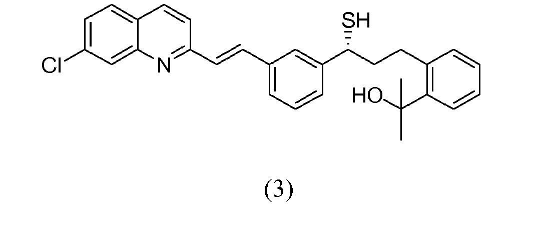
e) treating a mixture of the compound of formula (3) in an organic
solvent with camphorsulfonic acid of formula (4); and 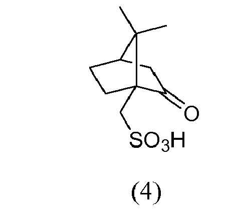
f) isolating the salt of formula (1).

4. The process according to claim 3 wherein the mixture resulting from step a) is maintained at a temperature comprised between 20 and 70 °C for a time period of 30 to 240 minutes and then cooled to a temperature comprised between -20 and 0 °C prior to the addition of the methylmagnesium halide.

5. The process according to anyone of claims 3 to 4 wherein R is a methyl group.

6. The process according to anyone of claims 3 to 5 wherein the methylmagnesium halide is methylmagnesium chloride.

7. The process according to anyone of claims 3 to 6 wherein the cerium (III) salt is CeCl₃.

8. Use of the salt of formula (1) in a process for the preparation of sodium montelukast.

9. Use according to claim 9 wherein the process comprises the following steps:
a) mixing the salt of formula (1) with an organic solvent;
b) optionally treating the mixture of step a) with a weak base;
c) adding (1-halomethyl-cyclopropyl)-acetic acid C₁-C₃ alkyl ester (17) and a strong base to the product obtained in step a) or b)
d) hydrolyzing the ester (10) obtained in step c) with a strong base;
e) acidifying the reaction mixture of step d)
f) treating the product (5) obtained in step e) with a primary or secondary organic amine;
g) isolating montelukast amine salt (19);
h) mixing the montelukast amine salt (19) with an organic solvent;
i) treating the product thus obtained with a source of sodium ion; and
j) isolating sodium montelukast (11).

10. The process according to claim 9, wherein the weak base used in step b) is NaHCO₃.

11. The process according to anyone of claims 9 to 10, wherein the strong base used in step c) is sodium methoxide.

12. The process according to anyone of claims 9 to 11, wherein the strong base used in step d) is sodium hydroxide.

13. The process according to anyone of claims 9 to 12, wherein the (1-halomethyl-cyclopropyl)-acetic acid C₁-C₃ alkyl ester is (1-bromomethyl-cyclopropyl)-acetic acid methyl ester.

14. The process according to anyone of claims 9 to 13, wherein the secondary organic amine is diisobutylamine.

15. The process according to anyone of claims 9 to 14, wherein the source of sodium ion is sodium tert-pentoxide.
